# EUROPÄISCHE PATENTSCHRIFT

(11) **EP 0 790 234 B1**
(45) Veröffentlichungstag und Bekanntmachung des Hinweises auf die Patenterteilung: **25.08.1999**
(21) Anmeldenummer: 97810070.9
(22) Anmeldetag: 06.02.1997
(51) Int. Cl.: C07C 253/00, C07C 253/20

(54) **Herstellung aromatischer Nitrile**
Process for the preparation of aromatic nitriles
Procédé de préparation de nitriles aromatiques

(30) Priorität: 15.02.1996 CH 39096
(43) Veröffentlichungstag der Anmeldung: 20.08.1997
(73) Patentinhaber: Ciba Specialty Chemicals Holding Inc., 4057 Basel (CH)
(72) Erfinder: Breitschuh, Richard, 14109 Berlin (DE)

(56) Entgegenhaltungen:
- EP-A- 0 080 700
- EP-A- 0 550 762
- EP-A- 0 609 179
- EP-A- 0 731 086
- DD-A- 117 872

## Beschreibung

Die vorliegende Anmeldung betrifft die Herstellung von aromatischen Nitrilen durch Umsetzung entsprechender Aldehyde mit Hydroxylaminsulfat in einem mit Wasser nicht mischbaren organischen Lösungsmittel in Anwesenheit einer tertiären Aminbase.

Die Umsetzung von Aldehyden mit Hydroxylaminsalzen und anschliessender Dehydratisierung des erhaltenen Oxims zum Nitril ist seit langem bekannt. Zur Dehydratisierung wurden verschiedene Methoden vorgeschlagen, so. z.B. in C.A. 85, 93176e (1976) durch Erhitzen in Dimethylformamid, in Synthesis 1979, 2, 112-113 und in Huaxue Shiji 1990, 12(5), 314, 292 durch Erhitzen in Ameisensäure, in Journal of Nanjing Univ. 1990, 26(2), 263-266, durch Erhitzen in Ameisensäure oder Eisessig und in J. Chem. Soc. 1933, IX, 43 durch Erhitzen in Essigsäureanhydrid. Von einer Verwendung von Dimethylformamid im grosstechnischen Massstab ist heutzutage aus toxischen und umweltpolitischen Ueberlegungen wenn möglich abzusehen. Das Arbeiten mit Ameisensäure führt zu sehr guten Resultaten, ist aber wegen der starken ätzenden Wirkung, der Toxizität und der aufwendigen Regenerierung nicht ratsam.

Gemäss einer im US-Patent 5 349 103 vorgeschlagenen Methode, können diese Nachteile durch Ersatz der Ameisensäure mit Propionsäure vermieden werden, wobei gleichwohl eine zufriedenstellende Ausbeute erzielt wird. Die mit sich bildendem Wasser abdestillierte Propionsäure kann regeneriert werden, allerdings in einem separaten Arbeitsgang.

Eine Arbeitsweise mit einem Hydroxylammoniumchlorid/Pyridin/Toluol-System wird in Synthesis 1982, 190 beschrieben. Bei diesem Verfahren bildet sich sublimierendes Pyridiniumchlorid, welches die Isolierung des gewünschten Endproduktes erheblich erschwert. Gemäss EP-B 80700 wird die Dehydratisierung des Oxims zum Nitril durch azeotropes Abdestilieren des Wassers mit Hilfe eines ein azeotropes Gemisch bildenden und mit Wasser nicht mischbaren Lösungsmittel bewerkstelligt. Die Ausbeuten sind dabei nicht immer befriedigend.

Es ist nun gefunden worden, dass die Umsetzung von aromatischen Aldehyden mit Hydroxylaminsulfat gegebenenfalls in einem ein azeotropes Gemisch bildenden und mit Wasser nicht mischbaren Lösungsmittel in Anwesenheit einer tertiären Aminbase, überraschenderweise sehr gute Ausbeuten an Nitril liefert, wobei das Nitril durch Phasentrennung problemlos vom entstandenen Pyridiniumhydrogensulfat gereinigt werden kann und das eventuell vorhandene Lösungsmittel durch Destillation während des Reaktionsprozesses direkt recycliert werden kann, was aus umweltpolitischen Ueberlegungen von grosser Bedeutung ist.

Die vorliegende Erfindung betrifft demnach ein Verfahren zur Herstellung von Nitrilen der Formel 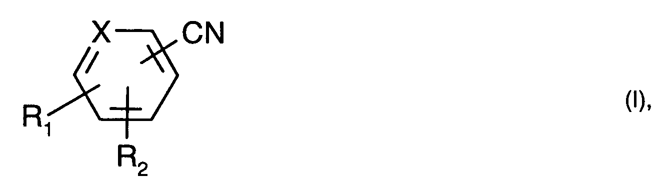 worin X CH oder N bedeutet und R₁ und R₂ unabhängig voneinander Wasserstoff, Chlor, Brom, C₁-C₆-Alkyl, C₁-C₆-Alkoxy, C₁-C₆-Alkylamino, CN, Phenyl, Phenoxy, Dimethylamino, Piperidyl, Morpholinyl oder Pyrrolidyl sind oder R₁ und R₂ zusammen einen ankondensierten Benzolring bilden,
durch Umsetzung eines Aldehyds der Formel 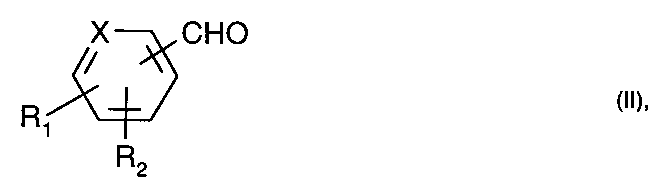 worin X, R₁ und R₂ die oben angegebene Bedeutung haben, mit Hydroxylaminsulfat und nachfolgender Dehydratisierung, dadurch gekennzeichnet, dass die Umsetzung in Gegenwart einer tertiären Aminbase der Formel 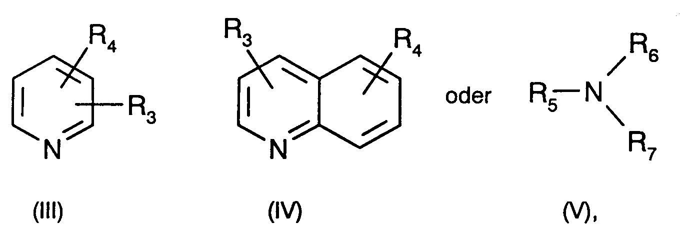 worin R₃ und R₄ unabhängig voneinander Wasserstoff, Methyl oder Ethyl, R₅ verzweigtes C₃-C₅-Alkyl oder Phenyl und R₆ und R₇ Methyl oder Ethyl bedeuten,
bei Temperaturen zwischen 100 und 160°C, bevorzugt zwischen 120 und 150°C, unter gleichzeitigem Abdestillieren des freiwerdenden Reaktionswassers bei einem Druck zwischen 0,02 und 1,5 bar, bevorzugt zwischen 0,1 bar und Atmosphärendruck, durchgeführt wird, anschliessender Entfernung der Ammoniumsalze, z.B. durch Phasentrennung, mit oder ohne vorherigem Zusatz von Wasser, und Isolierung des erhaltenen Nitrils nach allgemein üblichen Methoden.

Die Umsetzung erfolgt nach dem Reaktionsschema:

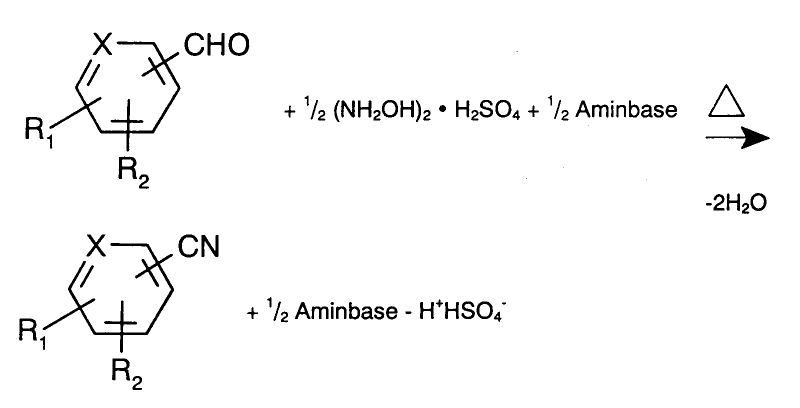

Zweckmässigerweise erfolgt die Umsetzung in Gegenwart eines ein azeotropes Gemisch bildenden und mit Wasser nicht mischbaren, vorzugsweise im Bereich zwischen 100 und 170°C siedenden organischen Lösungsmittels, wie z.B. Heptan, Octan, Methylcyclohexan, Cumol, Benzol und insbesondere Toluol.

R₁ und R₂ bedeuten als C₁-C₆-Alkyl, z.B. Methyl, Ethyl, n-Propyl, Isopropyl, n-Butyl, sec.-Butyl, tert.-Butyl, n-Pentyl, tert.-Amyl oder n-Hexyl und als C₁-C₆-Alkoxy z.B. Methoxy, Ethoxy, Isopropoxy, n-Butoxy, tert.-Butoxy, tert.-Amyloxy oder Hexyloxy.

Als C₁-C₆-Alkylamino bedeuten R₁ und R₂ z.B. Methylamino, Ethylamino, Isopropylamino, tert.-Butylamino, n-Pentylamino, Hexylamino.

R₅ bedeutet als verzweigtes C₃-C₆-Alkyl z.B. Isopropyl, tert.-Butyl oder tert.-Amyl.

Beispiele von tertiären Aminbasen der Formel III sind Pyridin, 2-Picolin, 2,6-Lutidin, 5-Ethyl-2-methylpyridin, der Formel IV z.B. Chinaldin und der Formel V z.B. N,N-Dimethylanilin, N,N-Diethylanilin oder Dimethylisopropylamin. Bevorzugt werden die Aminbasen der Formel III sowie Dimethyl- und Diethylanilin. Besonders bevorzugt sind Dimethylanilin und insbesondere Pyridin.

Von besonderem Interesse ist das erfindungsgemässe Verfahren zur Herstellung von Nitrilen der Formel I, worin X CH bedeutet, R₂ Wasserstoff ist, R₁ Wasserstoff, C₁-C₄-Alkyl oder Phenyl bedeutet und in Parastellung zur Cyangruppe steht.

Bei den Aldehyden der Formel II handelt es sich um bekannte Verbindungen. Sollten einige davon noch neu sein, so können sie in Analogie zu allgemein bekannten Methoden hergestellt werden.

Aus dem bei der Reaktion entstandenen Aminbase-H⁺HSO₄⁻-Salz kann durch Zugabe einer Base z.B. Natronlauge, die Aminbase zur erneuten Verwendung recycliert werden.

Das Hydroxylaminsulfat wird annähernd in stöchiometrischen Mengen bevorzugt aber stöchiometrisch oder im leichten Ueberschuss, d.h. 0,50 bis 0,58 Mol auf 1 Mol Aldehyd eingesetzt.

Die Aminbase wird zweckmässig in einer Menge von 1,0 bis 1,5 Mol auf 1 Mol Hydroxylaminsulfat eingesetzt.

Die durch das erfindungsgemässe Verfahren erhältlichen Nitrile sind wertvolle Zwischenprodukte u.a. für die Herstellung der nunmehr auch in der Literatur, z.B. in Colour Index, als DPP-Pigmente bezeichneten und seit einigen Jahren bewährten Diketopyrrolopyrrol-Pigmente.

Die nachfolgenden Beispiele erläutern die Erfindung.

Beispiel 1: Zu 735,7 g (6 mol) 4-Methyl-benzaldehyd, 81 g Wasser, 286,2 g (3,6 mol) Pyridin und 720 g Toluol werden bei 35-60°C insgesamt 502,3 g (3,0 mol) Hydroxylaminsulfat innerhalb von 1,5-2 Stunden in 4 Portionen so zugegeben, daß die nachfolgende Zugabe erst erfolgt, wenn das vorher zugegebene Hydroxylaminsulfat völlig gelöst ist. Nun werden weitere 720 g Toluol zugegeben und das Reaktionsgemisch zum Rückfluß am Wasserabscheider aufgeheizt. Bei ca. 95°C beginnt die Reaktionsmasse zu sieden und erreicht nach dem Ausdestillieren von ca. 200 ml Wasser eine Innentemperatur von 118°C. Das Reaktionsgemisch wird solange am Wasserabscheider erhitzt, bis 240-250 ml Wasser abdestilliert wurden. Nun wird die Hälfte des Toluols abdestilliert und anschließend bei einer Innentemperatur von nunmehr 126-129°C wiederum Wasser ausdestilliert, bis die Wasserabscheidung deutlich nachläßt (Gesamtdauer des Wasserabscheidens und der Toluol-Destillation: ca. 8 Stunden). Es wird auf 70-80°C abgekühlt, die untere Phase (Pyridiniumsalze) abgetrennt, die obere Phase einmal mit 250-300 ml Wasser gewaschen und bei zuletzt 120°C / 50 mbar die Wasserreste und das Toluol abdestilliert. Es verbleiben 641 g (89 % der Theorie) blaßgelbes geschmolzenes 4-Methyl-benzonitril (Sdp: 119°C bei 40 mbar) mit einer Reinheit von über 97 % (HPLC)*. Das Nitril kann durch anschließende Destillation weiter gereinigt werden.
* HPLC = High Performance Liquid Chromatography

Beispiel 2: Zu 490,6 g (4 mol) 4-Methyl-benzaldehyd und 19,8 g (0,25 mol) Pyridin werden 20,0 g (0,2 mol) 98%ige Schwefelsäure zugetropft und das so erhaltene Gemisch anschließend erhitzt. Bei 130-140°C und 180 mbar werden nun in kleinen Portionen insgesamt 197,8 g (2,5 mol) Pyridin und 334,9 g (2,0 mol) Hydroxylaminsulfat über einen Zeitraum von ca. 5 Stunden zugegeben wobei das Reaktionswasser abdestilliert, das mitgerissene Aldehyd/Nitril-Gemisch aber zurückgeführt wird. Nach Zugabe der letzten Portion wird noch eine weitere Stunde bei 130-140°C / 100 mbar erhitzt, auf 70-80°C abgekühlt und die untere Phase (geschmolzene Pyridiniumsalze) abgetrennt. Das verbleibende Rohprodukt wird noch einmal mit 100 ml Wasser gewaschen, bei 116°C/40 mbar die Wasserreste ausdestilliert und anschließend bei zuletzt 115°C/ 28 mbar 368,0 g farbloses 4-Methyl-benzonitril (78 % der Theorie) abdestilliert, das bei 28°C erstarrt. Die zuvor abgetrennte Schmelze von Pyridiniumsulfat und -hydrogensulfat wird mit 400 g Wasser verdünnt und mit 548 g (4,11 mol) 30% Natriumhydroxid auf pH = 8,5 gestellt. Nach Abtrennung der unteren Phase, die aus Natriumsulfat und Wasser besteht, verbleiben 288 g Pyridin mit einem Wassergehalt von 25-30%. Dieses kann ohne weitere Reinigung für den nächsten Ansatz wiederverwendet werden.

Beispiel 3: Zu 331 g (2 mol) 4-tert.-Butyl-benzaldehyd, 146,8 g (1,2 mol) 5-Ethyl-2-methylpyridin, 45 g Wasser und 250 g Toluol werden bei 40-60°C 167,5 g (1 mol) Hydroxylaminsulfat in 4 Portionen zugegeben. Nachdem alles Hydroxylaminsulfat gelöst ist werden 550 g Toluol zugefügt und das Gemisch zum Rückfluß am Wasserabscheider erhitzt. Nachdem 99 ml Wasser abgeschieden wurden, wird die Hälfte des Toluol abdestilliert wobei die Siedetemperatur von 119°C auf 126°C ansteigt, und solange Wasser ausdestilliert, bis weniger als 2 ml pro Stunde abgeschieden werden. Nun wird bei 50°C die untere Phase abgetrennt, die organische Phase mit dem Reaktionswasser in zwei Portionen gewaschen, die verbliebenen Wassermengen azeotrop ausdestilliert und das Toluol bei zuletzt 136°C/50 mbar ausdestilliert. Es verbleiben als Rückstand 295 g (88 % der Theorie) 4-tert-Butyl-benzonitril als goldbraunes Öl mit einem Gehalt von 95 % (HPLC)*, das destillativ noch weiter gereinigt werden kann (Sdp = 120°C/10 mbar).
* HPCL = High Performance Liquid Chromatography

Beispiel 4: Zu 662 g (4 mol) 4-tert.-Butyl-benzaldehyd, 292,3 g (2,4 mol) N,N-Dimethylanilin, 90 g Wasser und 800 g Xylol werden bei 60-90°C in zwei Portionen 335 g (2 mol) Hydroxylaminsulfat zugegeben und anschließend das Reaktionsgemisch zum Rückfluß am Wasserabscheider aufgeheizt. Nachdem ca. 210 ml Wasser abgeschieden wurden, wird auf 70°C abgekühlt, das zuvor abdestillierte Wasser zugegeben, die untere, wäßrige Phase abgetrennt, die organischen Phase noch einmal mit 100 ml Wasser gewaschen und bei zuletzt 150°C/50 mbar das Xylol abdestilliert. Es verbleiben 625,4 g (86 % der Theorie) von 88 %igem (HPLC)* 4-tert.-Butyl-benzonitril. Zur weiteren Reinigung kann entweder das beim Abkühlen auskristallisierende Amid abfiltriert oder das Nitril durch Destillation weiter gereinigt werden.

Beispiel 5: In 800 g Xylol, 45 g Wasser und 111,8 g (1,2 mol) 2-Picolin werden 372 g (2 mol) 4-Phenyl-benzaldehyd bei 50°C vorgelegt und in 4 Portionen mit 167,5 g (1 mol) Hydroxylaminsulfat bei 50-65°C versetzt. Nun wird zum Rückfluß am Wasserabscheider erhitzt und bis nach 6-7 Stunden die Wasserabscheidung deutlich nachläßt. Nun wird auf ca. 100°C abgekühlt, die zuvor abdestillierten 114 ml Wasser zugegeben und die untere Phase (Wasser + Picolinsalze) bei ca. 90°C abgetrennt. Die organische Phase wird noch einmal mit 100 ml Wasser bei 90°C gewaschen, die Produktlösung klärfiltriert und bei zuletzt 150°C / 70 mbar das Xylol abdestilliert. Es verbleiben als Rückstand 318,4 g (81 % der Theorie) einer hellbraunen Schmelze von 4-Phenyl-benzonitril mit einem analytischen Gehalt von 91 % (HPLC)*, die bei 80-85°C erstarrt.
* HPCL = High Performance Liquid Chromatography

## Patentansprüche

1. Verfahren zur Herstellung von Nitrilen der Formel 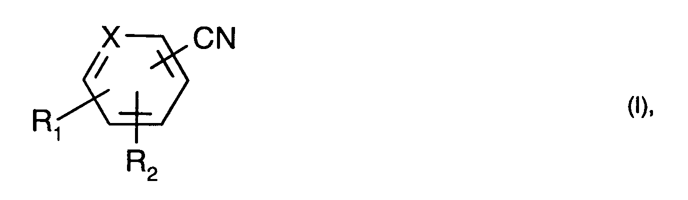 worin X CH oder N bedeutet und R₁ und R₂ unabhängig voneinander Wasserstoff, Chlor, Brom, C₁-C₆-Alkyl, C₁-C₆-Alkoxy, C₁-C₆-Alkylamino, CN, Phenyl, Phenoxy, Dimethylamino, Piperidyl, Morpholinyl oder Pyrrolidyl sind oder R₁ und R₂ zusammen einen ankondensierten Benzolring bilden,
durch Umsetzung eines Aldehyds der Formel 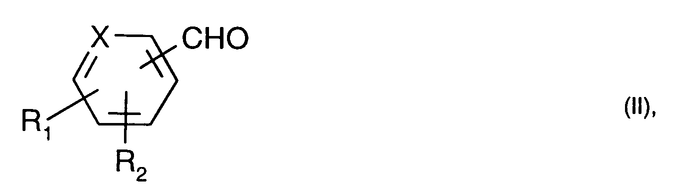 worin X, R₁ und R₂ die oben angegebene Bedeutung haben, mit Hydroxylaminsulfat und nachfolgender Dehydratisierung, dadurch gekennzeichnet, dass die Umsetzung in Gegenwart einer tertiären Aminbase der Formel 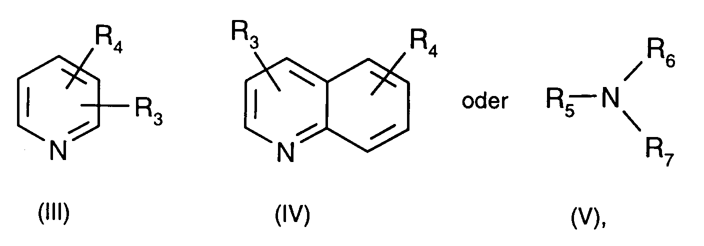 worin R₃ und R₄ unabhängig voneinander Wasserstoff, Methyl oder Ethyl, R₅ verzweigtes C₃-C₅-Alkyl oder Phenyl und R₆ und R₇ Methyl oder Ethyl bedeuten,
bei Temperaturen zwischen 100 und 160°C, bevorzugt zwischen 120 und 150°C, unter gleichzeitigem Abdestillieren des freiwerdenden Reaktionswassers bei einem Druck zwischen 0,02 und 1,5 bar, bevorzugt zwischen 0,1 bar und Atmosphärendruck, durchgeführt wird, anschliessender Entfernung der Ammoniumsalze und Isolierung des erhaltenen Nitrils nach allgemein üblichen Methoden.

2. Verfahren gemäss Anspruch 1, dadurch gekennzeichnet, dass die Umsetzung in Gegenwart eines ein azeotropes Gemisch bildenden und mit Wasser nicht mischbaren organischen Lösungsmittels durchgeführt wird.

3. Verfahren gemäss Anspruch 2, dadurch gekennzeichnet, dass als Lösungsmittel Chlorbenzol oder Toluol verwendet werden.

4. Verfahren gemäss Anspruch 1, dadurch gekennzeichnet, dass die Entfernung der Ammoniumsalze durch Phasentrennung, mit oder ohne vorherigem Zusatz von Wasser, erfolgt.

5. Verfahren gemäss Anspruch 1, dadurch gekennzeichnet, dass eine tertiäre Aminbase ausgewählt aus der Gruppe bestehend aus Pyridin, 2-Picolin, 2,6-Lutidin, 5-Ethyl-2-methylpyridin, Chinaldin, N,N-Dimethylamin, N,N-Diethylanilin und Dimethylisopropylamin verwendet wird.

6. Verfahren gemäss Anspruch 5, dadurch gekennzeichnet, dass Pyridin oder Dimethylamin verwendet wird.

7. Verfahren gemäss Anspruch 1 zur Herstellung von Nitrilen der Formel I, worin X CH bedeutet, R₂ Wasserstoff ist, R₁ Wasserstoff, C₁-C₄-Alkyl oder Phenyl bedeutet und in Parastellung zur Cyangruppe steht.

8. Verfahren gemäss Anspruch 1, dadurch gekennzeichnet, dass das Hydroxylaminsulfat in einer Menge von 0,5 bis 0,58 mol auf 1 Mol Aldehyd eingesetzt wird.

9. Verfahren gemäss Anspruch 8, dadurch gekennzeichnet, dass die Aminbase in einer Menge von 1,0 bis 1,5 mol auf 1 Mol Hydroxylaminsulfat eingesetzt wird.

## Claims

1. A process for the preparation of a nitrile of formula 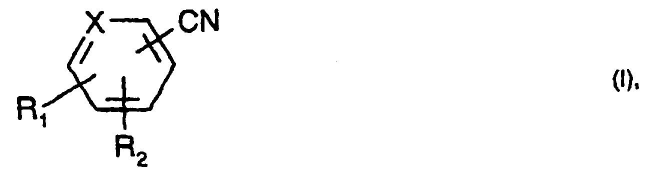 wherein X is CH or N and R₁ and R₂ are each independently of the other hydrogen, chloro, bromo, C₁-C₆alkyl, C₁-C₆alkoxy, C₁-C₆alkylamino, CN, phenyl, phenoxy, dimethylamino, piperidyl, morpholinyl or pyrrolidyl, or R₁ and R₂ together form a fused-on benzene ring,
by reacting an aldehyde of formula 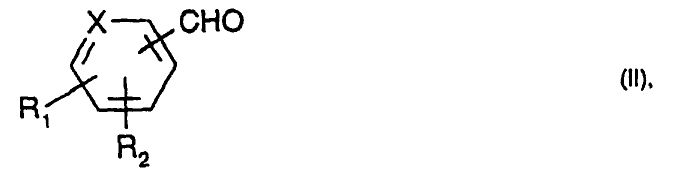 wherein X, R₁ and R₂ have the meaning given above, with hydroxylamine sulfate and with subsequent dehydration, which comprises carrying out the reaction in the presence of a tertiary amine base of formula 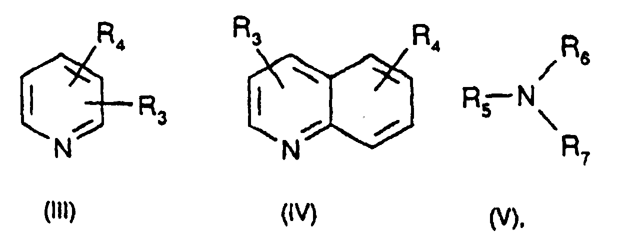 wherein R₃ and R₄ are each independently of the other hydrogen, methyl, or ethyl, R₅ is branched C₃-C₅alkyl or phenyl, and R₆ and R₇ are methyl or ethyl,
at temperatures from 100 to 160°C, preferably from 120 to 150°C, while distilling the released water of reaction off concomitantly at a pressure from 0.02 to 1.5 bar, preferably from 0.1 bar to atmospheric pressure, with subsequent removal of the ammonium salts, and isolating the resultant nitrile by customary methods.

2. A process according to claim 1, which comprises carrying out the reaction in the presence of a water-immiscible organic solvent forming an azeotropic mixture.

3. A process according to claim 2, wherein the solvent used is chlorobenzene or toluene.

4. A process according to claim 1, which comprises removing the ammonium salts by phase separation with or without prior addition of water.

5. A process according to claim 1, which comprises using a tertiary amine base selected from the group consisting of pyridine, 2-picoline, 2,6-lutidine, 5-ethyl-2-methylpyridine, quinaldine, N,N-dimethylamine, N,N-diethylaniline, and dimethylisopropylamine.

6. A process according to claim 5, which comprises using pyridine or dimethylamine.

7. A process according to claim 1 for the preparation of a nitrile of formula 1, wherein X is CH, R₂ is hydrogen, and R₁ is hydrogen, C₁-C₄alkyl and is in para position to the cyano group.

8. A process according to claim 1, wherein the hydroxylamine sulfate is used in an amount of 0.5 to 0.58 mol per 1 mol of aldehyde.

9. A process according to claim 8, wherein the amine base is used in an amount of 1.0 to 1.5 mol per 1 mol of hydroxylamine sulfate.

## Revendications

1. Procédé pour la préparation de nitriles de formule 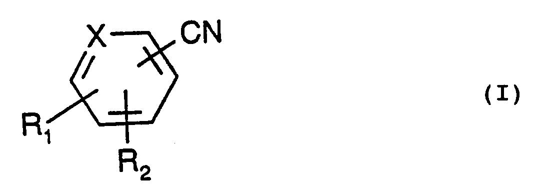 dans laquelle X représente les groupes CH ou N, et R₁ et R₂ représentent, indépendamment l'un de l'autre, des atomes d' hydrogène, de chlore, de brome, des groupes alkyle en C₁-C₆, alkoxy en C₁-C₆, (alkyl en C₁-C₆)amino, CN, phényle, phénoxy, diméthylamino, pipéridyle, morpholinyle ou pyrrolidyle, ou R₁ et R₂ forment ensemble un noyau benzénique condensé,
par transformation d'un aldéhyde de formule 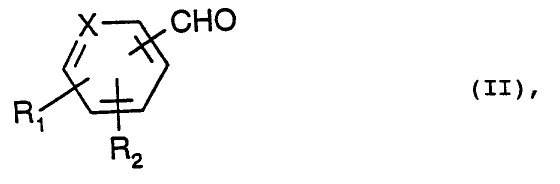 dans laquelle X, R₁ et R₂ ont les significations données ci-dessus, avec le sulfate d'hydroxylamine et une déshydratation ultérieure, caractérisé en ce que l'on réalise la réaction en présence d'une basé aminée tertiaire de formule 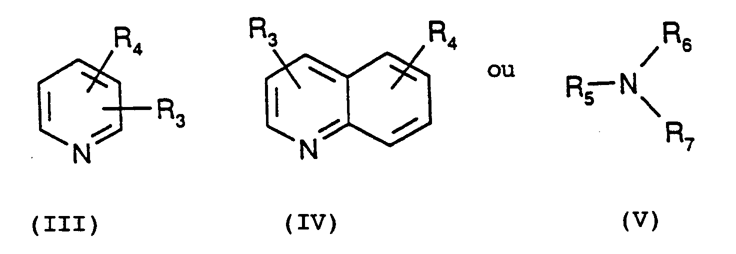 où R₃ et R₄ représentent, indépendamment l'un de l'autre, un atome d'hydrogène, des groupes méthyle ou éthyle, R₅ représente un groupe alkyle en C₃-C₅ ramifié ou phényle, et R₆ et R₇ représentent un groupe méthyle ou éthyle,
à une température comprise entre 100 et 160°C, de préférence entre 120 et 150°C, en chassant simultanément par distillation l'eau réactionnelle libérée sous une pression comprise entre 0,02 et 1,5 bar, de préférence entre 0,1 bar et la pression atmosphérique, par élimination ultérieure des sels d'ammonium, par exemple par séparation de phases, avec ou sans ajout préalable d'eau, et par isolement du nitrile obtenu selon des méthodes généralement connues.

2. Procédé selon la revendication 1, caractérisé en ce que l'on met en oeuvre la réaction en présence d'un solvant organique formant un mélange azéotropique et non miscible à l'eau.

3. Procédé selon la revendication 2, caractérisé en que l'on utilise le chlorobenzène ou le toluène en tant que solvant.

4. Procédé selon la revendication 1, caractérisé en ce que l'on met en oeuvre l'élimination des sels d'ammonium par séparation de phases avec ou sans ajout préalable d'eau.

5. Procédé selon la revendication 1, caractérisé en ce que l'on emploie une base aminée tertiaire prise dans un groupe comportant la pyridine, la 2-picoline, la 2,6-lutidine, le 5-éthyl-2-méthylpyridine, la quinaldine, la N,N-diméthylamine, la N,N-diéthylaniline et la diméthylisopropylamine.

6. Procédé selon la revendication 5, caractérisé en ce que l'on emploie la pyridine ou la diméthylamine.

7. Procédé selon la revendication 1 pour la préparation de nitriles de formule I, où X représente un groupe CH, R₂ représente un atome d'hydrogène, R₁ représente un atome d'hydrogène, des groupes alkyle en C₁-C₄ ou phényle et se trouve en position para par rapport au groupe cyano.

8. Procédé selon la revendication 1, caractérisé en ce que l'on emploie le sulfate d'hydroxylamine en une quantité de 0,5 à 0,58 mole par mole d'aldéhyde.

9. Procédé selon la revendication 8, caractérisé en ce que l'on emploie la base aminée en une quantité de 1,0 à 1,5 mole par mole de sulfate d'hydroxylamine.
